# EUROPEAN PATENT APPLICATION

(11) **EP 1 733 750 A1**
(43) Date of publication of application: **20.12.2006**
(21) Application number: 06115405.0
(22) Date of filing: 13.06.2006
(51) Int. Cl.: A61M 5/34, A61M 5/50

(54) **Syringe barrel and syringe using the same**

(30) Priority: 14.06.2005 US 151580
(71) Applicant: HUANG, Hung-Chi, Taichung City (TW)
(72) Inventor: HUANG, Hung-Chi, Taichung City (TW)
(74) Representative: Viering, Jentschura & Partner

(57) **Abstract**

A syringe barrel (10) and syringe uses the same wherein the syringe barrel comprises a round plastic bracket at the front thereof, which defines a needle hub (11); the bracket is formed at the same time when the barrel (10) is molded. The bracket is further covered by a rubber flexible member (17) through injection molding. The radius of the inner rim of the flexible member (17) is smaller than that of the outer radius of a needle mount (151), whereby the needle mount will be coupled tightly with the needle hub (11) using the bracket of the barrel (10).

## Description

### Field of the Invention:

The present invention relates to syringe barrels, more particularly to a syringe barrel and syringe uses the same wherein a needle mount and a needle hub of the syringe will be coupled with precision.

### Background of the Invention:

A safety syringe of the prior art is characterized by a syringe barrel capable of pulling a needle back therein after an injection, so as to prevent reusing it. At the same time, the syringe is safer to abandon with the needle hidden.

Accordingly, the safety syringe of the prior art comprises a syringe barrel, a push rod, a needle hub and a syringe needle. The needle hub is mounted on the front face of the barrel for housing a needle mount. The connection of the needle mount into the needle hub should be at one hand tight, at the other hand retractable, so that the needle can be pulled back into the barrel by the push rod. To achieve the objective, the rear end of the needle mount where the needle hub is engaged is provided with a receptacle hole of large radius. The receptacle hole is further provided with a neck section, and the front terminal of the push rod has a hooked conic head. Thereby, as the push rod inject a solution out of the barrel, the hooked conic head will be embedded into the receptacle hole and hooked with the needle mount. As the push rod id pulled backward, the needle will also be pulled into the barrel.

However, the needle mount of the prior art should be made with precision so as to assure the pulling back of the needle, which is a difficult manufacturing task and therefore expensive. Further, to assure the pulling back of the needle, the drag between the needle mount and the barrel must be lessened, therefore sacrificing the tightness of the connection. As a result, the needle structure is likely to let go if the drag of a human body is too large, invalidating an injection. It is a further disadvantage that the solution in the barrel may leak through where the needle mount and the needle hub of the barrel, leading to a drop of the needle.

On the other hand, if the connection between of needle mount and the needle hub is too tight, the conic head of the push rod will depart from the receptacle hole as it is being pulled back. Therefore, the purpose of safety use cannot be attained.

To summarize, the conventional needle mount made of hard plastic materials needs a high-precision injection molding to solve the above dilemma, which is expensive to the manufacturer and therefore the cost of the safety syringes is high. Further, the needle hub attached to the syringe barrel could be slightly deformed due to high temperature in the storage space, which is another factor influences the required precision.

### Summary of the Invention:

Accordingly, the syringe barrel and syringe uses the same wherein the syringe barrel comprises a round plastic bracket at the front end of the barrel, which defines a needle hub; the bracket is formed at the same time when the barrel is molded. The bracket is further covered by a rubber flexible member through injection molding. The radius of the inner rim of the flexible member is smaller than that of the outer radius of a needle mount, whereby the needle mount will be coupled tightly with a needle hub using the bracket of the barrel. The elastic deformation of the inner space of the flexible member will provide the effect of pinching the needle mount, which achieves a precision coupling with a low cost.

The various objects and advantages of the present invention will be more readily understood from the following detailed description when read in conjunction with the appended drawings.

### Brief Description of the Drawings:

Fig.1 is side cross sectional view of a syringe of the present invention.
Fig.2 is local cross sectional view of the syringe in Fig.1.
Fig.3 is local perspective view of the syringe in Fig.1.
Fig.4 is local perspective view of the second preferred embodiment of the present invention.
Fig.5 is another local perspective view of a syringe and a needle hub of the present invention.
Fig.6 is a cross sectional view of the syringe and the needle hub in Fig.5.
Fig.7 is local perspective view of the second preferred embodiment of the present invention, showing the barrel and the needle hub.
Fig.8 is a cross sectional view of the syringe and the needle hub in Fig.7.

### Detailed Description of the Preferred Embodiments:

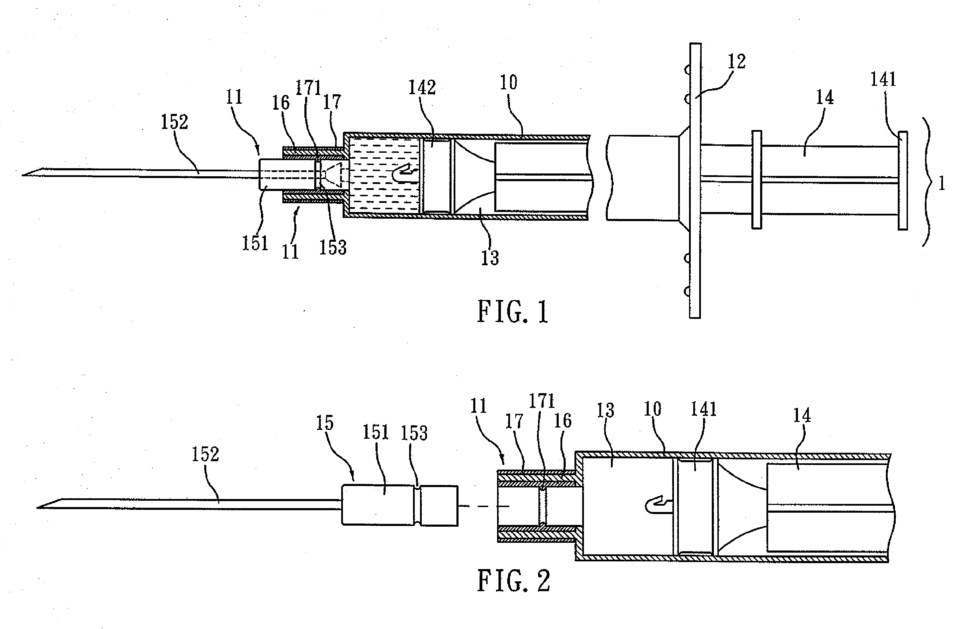

Referring to Figs.1 and 2, a syringe barrel according to the present invention comprises a barrel 10 being a hollow cylinder and a needle hub 1I of radius smaller than that of the barrel 10 extended from the front end of the barrel 10. The rear end of the barrel 10 is provided with a finger flange 12 which is an enlarged and extended portion thereof. The inner space of the barrel 10 defines a plunger receptacle 13 which is extended out of the barrel 10 from the rear end.

The syringe further includes a thumb rest 14 coupled within the plunger receptacle 13 of the barrel 10. At the front end of the thumb rest 14, there forms a stopper 141 taking the shape of a block of round cross section; at the rear end of the thumb rest 14, there forms a thumb support 142 bigger than the cross section of the plunger receptacle 13.

The syringe further includes a needle structure 15 comprising a round needle mount 151 being an elongated section and a needle cannula 152 being a long, hollow needle. The round needle mount 151 and the needle hub 11 form a stable connection as the round needle mount 151 is engaged with the annularly distributed bulges 1 on the inner wall of the needle hub 11. Further, one end of the needle mount 151 is provided with a recessed retaining groove 153.

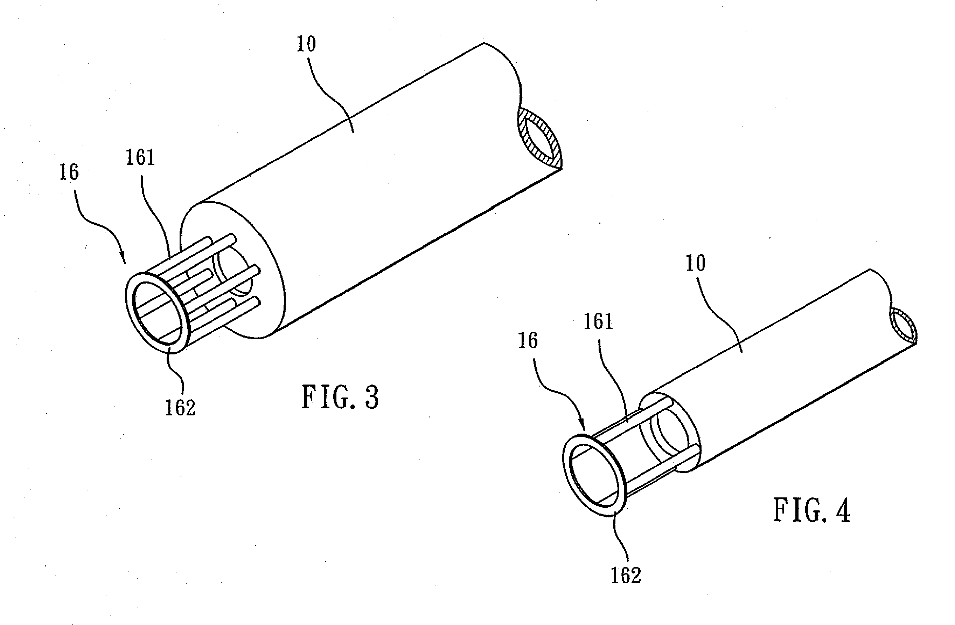

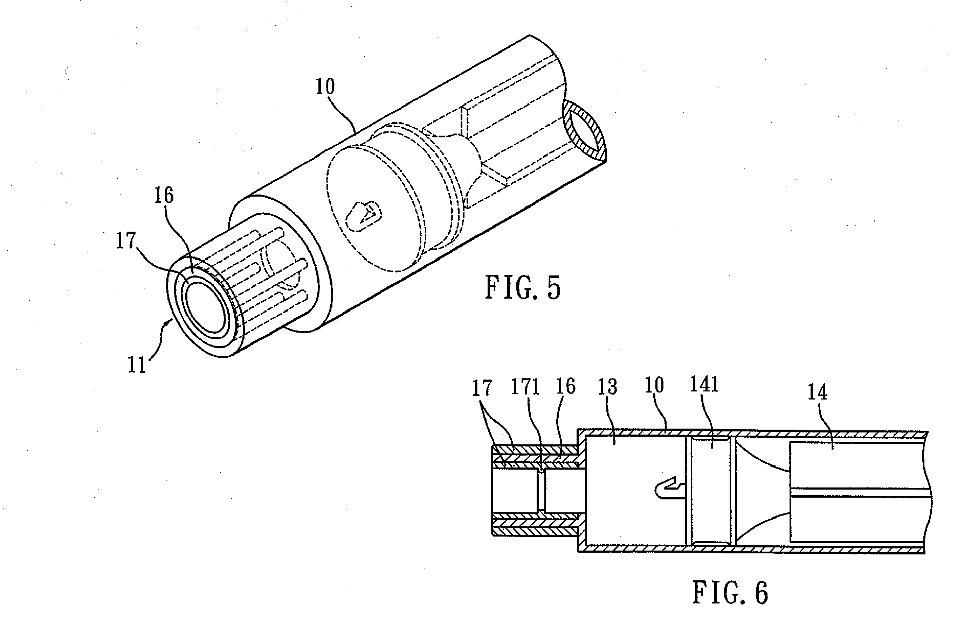

Referring to Figs.3, 5 and 6, a needle hub 11 of radius smaller than that of the barrel 10 is extended from the front end of the barrel 10. As the barrel 10 was first formed, the front end thereof is extended to form a plastic bracket 16, which is a bundle of parallel bulged columns 161. The free end of the collection of bulged columns 161 is provided with a round ring 162 connecting the columns 161, whereby the structure of the needle hub 11 is strengthened.

The outer layer of the bracket 16, which indicates the inner and outer rims thereon, is provided with a flexible member 17 made of plastic rubber. The connection of the flexible member 17 to the bracket 16 is done by injection coating or injecting a plastic sleeve corresponding to the inner and inner rims of the bracket 16 on the bracket 16. No matter which connection way, the plastic material of the flexible member 17 possess a property of varying the space therein, whereby the connection between the needle mount 151 and the needle hub 11 can be tight, achieving not only a high-precision connection but also a retractable coupling by a push rod. The latter function makes the syringe not reusable.

Further, the above-mentioned flexible member 17 has an inner radius less than that of the outer radius of the round needle mount 151. Since the inner e inner rim of the flexible member 17 forms an inner flange 171, the needle mount 151 will be retained by the flexible member 17 as the needle mount 151 inserted into the needle hub 11.

The present invention and the conventional needle hub and needle mount differ in that the latter is made of a hard plastic material. However, since that the bracket 16 is made of a hard plastic material and the flexible member 17 is made of flexible rubber, the inner space of the flexible member 17 is flexsible. Further, the inner radius of the flexible member 17 is slightly smaller than that of a needle mount 151, whereby the needle mount 151 will be erected with precision due to the flexibility of the flexible member 17.

This design has the following advantages. First, since the rubber material making the flexible member 17 is superior in changing the shape of its receptacle space, the tight connection between the needle mount and the needle hub will thus be assured. Thereby, the needle mount cannot be easily pulled out.

Second, to pull out the needle mount from the needle hub, a proper force exerted on the needle mount will suffice; an extremely large force is not necessary.

Third, the flex member around the inner rim of the needle hub can be implanted by regular injection molding, achieving a tight connection of the needle mount to the needle hub.

To be consistent, the bracket 16 is made of a plastic material, the same as that of the needle hub. Since the bracket 16 is formed at the same time the barrel 10 is being made, the connecting structure of needle hub is tough. Further, the bracket 16 is provided with a bundle of columns 161, forming a rib structure by which the friction between the mount and the hub is enhanced, achieving tight connection between the two.

The above structure is valid for syringes of 3-5 cc. Syringes less than 1 cc is not applicable, because the contact area between the needle mount and the needle hub is getting smaller. As the thumb support and the associated push rod are being pushed forward, most of the pressure will be concentrated on the bottom face of the mount, inevitably leading to a departure of the mount from the hub, since the pressure force exceeds the frictional drag.

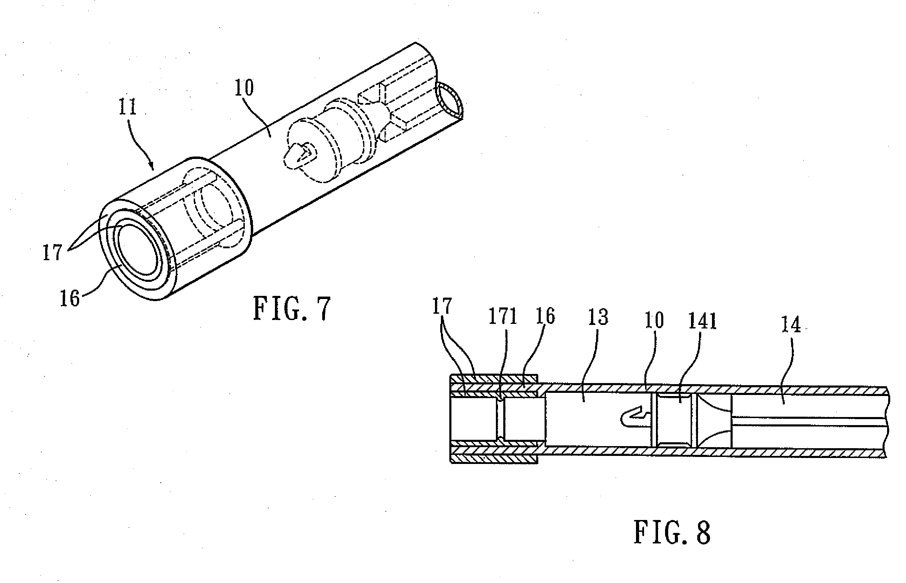

On the other hand, the present invention can be used to syringes less than 1 cc, as shown in Figs.4, 7 and 8. Although the contact area between the needle mount 151 and the needle hub 11 is getting smaller, the flexible member 17 of the hub 11 provides extra pinch to the needle mount 151 and therefore enhances the connection. The connection is further strengthened by the annular inner flange 171 within the flexible member 17 and, further, the retaining groove 153 therein.

The present invention is thus described, and it will be obvious that the same may be varied in many ways. Such variations are not to be regarded as a departure from the spirit and scope of the present invention, and all such modifications as would be obvious to one skilled in the art are intended to be included within the scope of the following claims.

## Claims

1. A syringe barrel for receiving a push rod, comprising:
a bracket at a front end of said syringe barrel formed together with said syringe barrel, said needle hub consisting of a plastic bracket and a flexible member covering said bracket.

2. The syringe barrel of claim 1 wherein said flexible member is made of a rubber material.

3. The syringe barrel of claim 2 wherein an inner rim of said rubber flexible member of said bracket is provided with an inner flange at a predetermined location.

4. The syringe barrel of claim 2 wherein said bracket consists of a bundle of annularly distributed parallel columns.

5. The syringe barrel 2 wherein said bracket is a single hollow cylindrical object.

6. The syringe barrel of claim 2 wherein said rubber flexible member is attached onto said bracket by injection molding.

7. The syringe barrel of claim 2 wherein said flexible member is a hollow sleeve corresponding to an inner rim and an outer rim of said bracket, thereby said flexible member being capable of being attached onto said bracket.

8. The syringe barrel of claim 4 wherein the intervals of said columns bulged into said bracket are respectively provided with grooves.

9. The syringe barrel claim 4 wherein the intervals of said columns bulged into said bracket are respectively provided with rib structures.

10. A syringe using said syringe barrel, comprising:
a syringe barrel being a hollow cylinder with a needle hub formed at a front end thereof, said needle hub further including a bracket extended from said syringe barrel and a flexible member covering said bracket;
a push rod with a thumb support installed at a rear open end of said syringe barrel, a rod section of said push rod being housed within said syringe barrel, a front end of said rod section of said push rod being provided with a round stopper, a rear end of said push rod being provided with a thumb sopport; and
a needle structure comprising a needle mount and a needle, said needle mount being inserted into said needle hub, said needle being inserted into said needle mount.

11. The syringe of claim 10 wherein said flexible member is made of a rubber material.

12. The syringe of claim 10 wherein an outer rim of said needle mount is provided with a retaining groove.

13. The syringe of claim 11 wherein an inner rim of said flexible member covering said bracket is provided with an inner flange.

14. The syringe of claim 11 wherein said bracket is a plurality of annually distributed, parallel columns bulged into said bracket.

15. The syringe of claim 11 wherein said bracket is a single hollow cylindrical object.

16. The syringe of claim 11 wherein said rubber flexible member is attached onto said bracket by injection molding

17. The syringe of claim 11 wherein said flexible member is a hollow sleeve corresponding to an inner rim and an outer rim of said bracket, thereby said flexible member being capable of being attached onto said bracket.

18. The syringe of claim 11 wherein said flexible member made of plastic rubber has said needle hub with a radius of an inner rim thereof slightly smaller than an outer radius of said needle mount.
